# EUROPEAN PATENT APPLICATION

(11) **EP 4 717 313 A2**
(43) Date of publication of application: **01.04.2026**
(21) Application number: 25213976.1
(22) Date of filing: 03.09.2020
(51) Int. Cl.: A61P 25/00

(54) **VAFIDEMSTAT FOR USE IN TREATING AUTISM SPECTRUM DISORDER**

(30) Priority: 03.09.2019 EP 19382751; 03.10.2019 EP 19382855
(62) Divisional of application: 24198301.4
(71) Applicant: Oryzon Genomics, S.A., 28014 Madrid (ES)
(72) Inventor: BUESA ARJOL, Carlos Manuel, 08940 Cornellà de Llobregat (ES); BULLOCK, Roger Alan, 08940 Cornellà de Llobregat (ES); RAMOS QUIROGA, José Antonio, 08035 Barcelona (ES)
(74) Representative: Vossius & Partner Patentanwälte Rechtsanwälte mbB

(57) **Abstract**

Provided herein is vafidemstat for use in a method of treating autism spectrum disorder.

## Description

### FIELD

The present invention relates to methods for treating autism spectrum disorder.

### BACKGROUND

Autism spectrum disorder (ASD) is a developmental disorder that affects communication and behavior. Although autism can be diagnosed at any age, it is said to be a "developmental disorder" because symptoms generally appear in the first two years of life. People with ASD have difficulty with communication and interaction with other people, restricted interests and repetitive behaviors, and symptoms that hurt the person's ability to function properly in school, work, and other areas of life.

There are no treatments approved by the FDA that address the core features of ASD. Currently approved therapies for use in ASD patients are indicated for the treatment of irritability associated with ASD and exhibit side-effects, including sedation and weight gain.

Thus, there is a strong and unmet medical need for new and/or improved drugs for treating ASD, particularly drugs that act via novel mechanisms of action and treat the core features of ASD, and/or with a more favorable side effect profile than current therapies. The present invention addresses these and other needs.

### SUMMARY OF THE INVENTION

The invention provides novel methods for treating autism spectrum disorder by using vafidemstat (or a pharmaceutically acceptable salt or solvate thereof).

Thus, the present invention provides vafidemstat, or a pharmaceutically acceptable salt or solvate thereof, for use in the treatment of autism spectrum disorder.

The present invention further provides a method for treating autism spectrum disorder in a patient (preferably a human), comprising administering to the patient a therapeutically effective amount of vafidemstat, or a pharmaceutically acceptable salt or solvate thereof.

The present invention further provides the use of vafidemstat, or a pharmaceutically acceptable salt or solvate thereof, for the manufacture of a medicament for the treatment of autism spectrum disorder.

The present invention further provides the use of vafidemstat, or a pharmaceutically acceptable salt or solvate thereof, for the treatment of autism spectrum disorder.

### BRIEF DESCRIPTION OF THE DRAWINGS

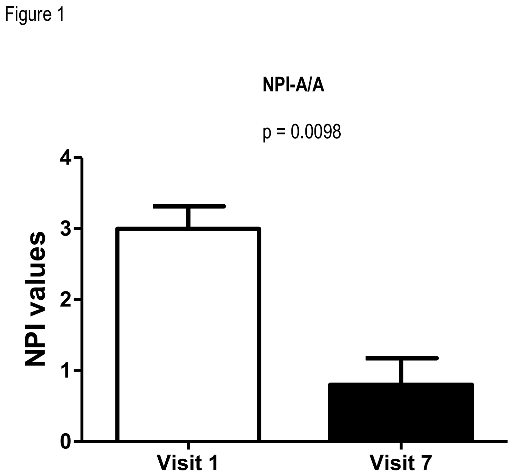

Figure 1 shows the effect of treatment with vafidemstat (as defined herein and in Example 1) to treat aggression in ASD patients, as shown by a statistically significant reduction in the Neuropsychiatric Inventory (NPI) 4-item agitation/aggression (NPI A/A) subscale from visit 1 (baseline, pre-treatment) to visit 7 (8 weeks treatment with vafidemstat), as described in more detail in Example 2. Data is represented as mean± standard error of the mean (SEM); p=0.0098.

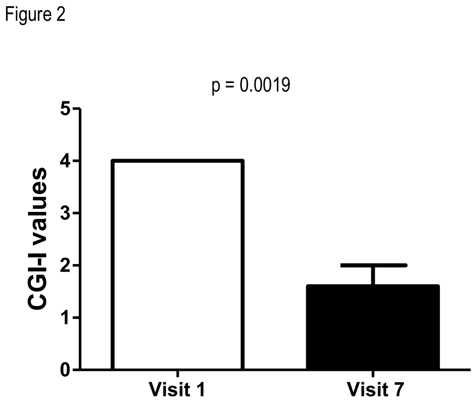

Figure 2 shows the effect of treatment with vafidemstat (as defined herein and in Example 1) to treat aggression in ASD patients, as shown by a statistically significant reduction in the Clinical Global Impression of Improvement (CGI-I) score from visit 1 (baseline, pre-treatment) to visit 7 (8 weeks treatment with vafidemstat), as described in more detail in Example 2. Data is represented as mean± standard error of the mean (SEM); p=0.0019.

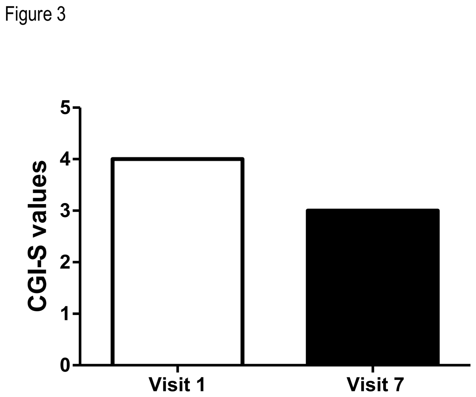

Figure 3 shows the effect of treatment with vafidemstat (as defined herein and in Example 1) to treat aggression in ASD patients, as shown by a reduction in the Clinical Global Impression of Severity (CGI-S) score from visit 1 (baseline, pre-treatment) to visit 7 (8 weeks treatment with vafidemstat), as described in more detail in Example 2. Data is represented as mean± standard error of the mean (SEM).

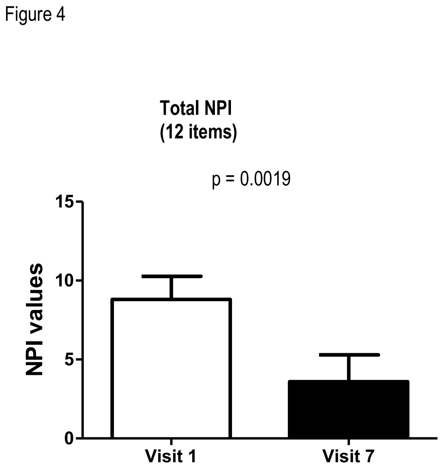

Figure 4 shows the efficacy of vafidemstat to treat ASD, as shown by a reduction in the Total NPI (12 items) score from visit 1 (baseline, pre-treatment) to visit 7 (8 weeks treatment with vafidemstat), as described in more detail in Example 2. Data is represented as mean± SEM; p=0.0019.

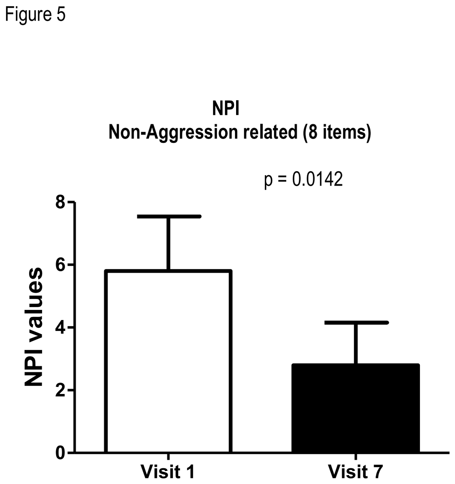

Figure 5 shows the efficacy of vafidemstat to treat ASD, as shown by a reduction in the NPI non-aggression related items (8 items) combined score from visit 1 (baseline, pre-treatment) to visit 7 (8 weeks treatment with vafidemstat), as described in more detail in Example 2. Data is represented as mean± SEM; p=0.0142.

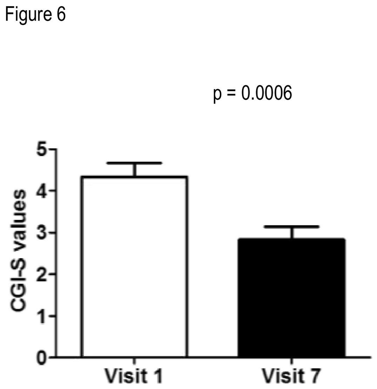

Figure 6 shows the effect of treatment with vafidemstat in the Clinical Global Impression of Severity (CGI-S) score from visit 1 (baseline, pre-treatment) to visit 7 (8 weeks treatment with vafidemstat) for a cohort of ASD patients including one additional patient, as described in more detail in Example 2.4. Data is represented as mean± standard error of the mean (SEM).

### DETAILED DESCRIPTION OF THE INVENTION

The invention is based on the unexpected finding that vafidemstat is useful and particularly well-suited as a therapeutic agent to treat ASD. Vafidemstat has been reported to be useful to reduce aggressiveness, such as aggressiveness associated with a disease, without sedative effects. Vafidemstat is currently in a Phase IIa clinical trial evaluating its efficacy in treating aggression in patients with ASD, attention deficit hyperactivity disorder (ADHD) and borderline personality disorder (BPD) (REIMAGINE trial). Results of this clinical trial unexpectedly demonstrated that vafidemstat is not only effective to treat aggression in ASD patients, but exhibits additional therapeutic effects on ASD, as detailed below and in the Examples. Vafidemstat is useful as a treatment for ASD, including adult ASD.

Accordingly, the present invention provides vafidemstat or a pharmaceutically acceptable salt or solvate thereof for use in the treatment of ASD.

The present invention further provides a method for treating ASD in a patient (preferably a human), comprising administering to the patient a therapeutically effective amount of vafidemstat or a pharmaceutically acceptable salt or solvate thereof.

The present invention further provides the use of vafidemstat or a pharmaceutically acceptable salt or solvate thereof for the manufacture of a medicament for the treatment of ASD.

The present invention further provides the use of vafidemstat or a pharmaceutically acceptable salt or solvate thereof for the treatment of ASD.

In some embodiments, the present invention provides vafidemstat or a pharmaceutically acceptable salt or solvate thereof for use in the treatment of ASD by treating (e.g. alleviating or improving) one or more core features of ASD.

In some embodiments, the present invention provides a method for treating ASD in a patient (preferably a human) by treating (e.g. alleviating or improving) one or more core features of ASD, the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of ASD by treating (e.g. alleviating or improving) one or more core features of ASD.

In some embodiments, the present invention provides the use of vafidemstat or a pharmaceutically acceptable salt or solvate thereof for the treatment of ASD by treating (e.g. alleviating or improving) one or more core features of ASD.

In accordance with the present invention, "core feature(s) of ASD" mean the essential features of ASD according to the Diagnostic and Statistical Manual of Mental Disorders, Fifth Edition (DSM-5), as published by the American Psychiatric Association; these include social communication and social interaction impairments and restricted, repetitive patterns of behavior or interests.

In some embodiments, the present invention provides vafidemstat or a pharmaceutically acceptable salt or solvate thereof for use in the treatment of ASD by treating (e.g. alleviating or improving) one or more non-aggressive symptoms of ASD.

In some embodiments, the present invention provides a method for treating ASD in a patient (preferably a human) by treating (e.g. alleviating or improving) one or more non-aggressive symptoms of ASD, the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of ASD by treating (e.g. alleviating or improving) one or more non-aggressive symptoms of ASD.

In some embodiments, the present invention provides the use of vafidemstat or a pharmaceutically acceptable salt or solvate thereof for the treatment of ASD by treating (e.g. alleviating or improving) one or more non-aggressive symptoms of ASD.

In accordance with the present invention, "non-aggressive" as for example used in the context of an ASD symptom means that said symptom of ASD is not directly related to or associated with aggression or aggressive behavior. "Aggression", "aggressive" and related terms, as used herein, refer to any kind of abnormal, pathological or inappropriate aggressive or violent behavior, hostility or agitation, for example physical or verbal, including interpersonal aggressiveness (i.e. towards other subjects) and/or intrapersonal aggressiveness (i.e. self-aggressiveness).

Examples of non-aggressive symptoms of ASD include: deficits in social-emotional reciprocity (e.g. abnormal social approach and failure of normal back-and-forth conversation, reduced sharing of interests, emotions, or affect, and failure to initiate or respond to social interactions); deficits in nonverbal communicative behaviors used for social interaction (e.g. poorly integrated verbal and nonverbal communication, abnormalities in eye contact and body language, deficits in understanding and use of gestures, or lack of facial expressions and nonverbal communication); deficits in developing, maintaining, and understand relationships (e.g. difficulties adjusting behavior to suit various social contexts, difficulties in sharing imaginative play or in making friends, or absence of interest in peers); stereotyped or repetitive motor movements, use of objects, or speech (e.g. simple motor stereotypes, lining up toys or flipping objects, echolalia, or idiosyncratic phrases); insistence on sameness, inflexible adherence to routines, or ritualized patterns of verbal or nonverbal behavior (e.g. extreme distress at small changes, difficulties with transitions, rigid thinking patterns, greeting rituals, need to take same route or eat same food every day); highly restricted, fixated interests that are abnormal in intensity or focus (e.g., strong attachment to or preoccupation with unusual objects, excessively circumscribed or perseverative interests); and hyper- or hyporeactivity to sensory input or unusual interest in sensory aspects of the environment (e.g. apparent indifference to pain/temperature, adverse response to specific sounds or textures, excessive smelling or touching of objects, visual fascination with lights or movement).

In some embodiments, the present invention provides vafidemstat or a pharmaceutically acceptable salt or solvate thereof for use in the treatment of ASD by treating (e.g. alleviating or improving) one or more core features of ASD and by treating (e.g. reducing) aggression.

In some embodiments, the present invention provides a method for treating ASD in a patient (preferably a human) by treating (e.g. alleviating or improving) one or more core features of ASD and by treating (e.g. reducing) aggression, the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of ASD by treating (e.g. alleviating or improving) one or more core features of ASD and by treating (e.g. reducing) aggression.

In some embodiments, the present invention provides the use of vafidemstat or a pharmaceutically acceptable salt or solvate thereof for the treatment of ASD by treating (e.g. alleviating or improving) one or more core features of ASD and by treating (e.g. reducing) aggression.

In some embodiments, the present invention provides vafidemstat or a pharmaceutically acceptable salt or solvate thereof for use in the treatment of ASD by treating (e.g. alleviating or improving) one or more non-aggressive symptoms of ASD and by treating (e.g. reducing) aggression.

In some embodiments, the present invention provides a method for treating ASD in a patient (preferably a human) by treating (e.g. alleviating or improving) one or more non-aggressive symptoms of ASD and by treating (e.g. reducing) aggression, the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of ASD by treating (e.g. alleviating or improving) one or more non-aggressive symptoms of ASD and by treating (e.g. reducing) aggression.

In some embodiments, the present invention provides the use of vafidemstat or a pharmaceutically acceptable salt or solvate thereof for the treatment of ASD by treating (e.g. alleviating or improving) one or more non-aggressive symptoms of ASD and by treating (e.g. reducing) aggression.

In some embodiments, the present invention provides vafidemstat or a pharmaceutically acceptable salt or solvate thereof for use in the treatment of ASD by treating (e.g. alleviating or improving) one or more core features of ASD and by treating (e.g. reducing) agitation.

In some embodiments, the present invention provides a method for treating ASD in a patient (preferably a human) by treating (e.g. alleviating or improving) one or more core features of ASD and by treating (e.g. reducing) agitation, the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of ASD by treating (e.g. alleviating or improving) one or more core features of ASD and by treating (e.g. reducing) agitation.

In some embodiments, the present invention provides the use of vafidemstat or a pharmaceutically acceptable salt or solvate thereof for the treatment of ASD by treating (e.g. alleviating or improving) one or more core features of ASD and by treating (e.g. reducing) agitation.

In some embodiments, the present invention provides vafidemstat or a pharmaceutically acceptable salt or solvate thereof for use in the treatment of ASD by treating (e.g. alleviating or improving) one or more non-aggressive symptoms of ASD and by treating (e.g. reducing) agitation.

In some embodiments, the present invention provides a method for treating ASD in a patient (preferably a human) by treating (e.g. alleviating or improving) one or more non-aggressive symptoms of ASD and by treating (e.g. reducing) agitation, the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of ASD by treating (e.g. alleviating or improving) one or more non-aggressive symptoms of ASD and by treating (e.g. reducing) agitation.

In some embodiments, the present invention provides the use of vafidemstat or a pharmaceutically acceptable salt or solvate thereof for the treatment of ASD by treating (e.g. alleviating or improving) one or more non-aggressive symptoms of ASD and by treating (e.g. reducing) agitation.

In some embodiments, the present invention provides vafidemstat or a pharmaceutically acceptable salt or solvate thereof for use in the treatment of ASD by treating (e.g. alleviating or improving) one or more core features of ASD and by treating (e.g. reducing) agitation and aggression.

In some embodiments, the present invention provides a method for treating ASD in a patient (preferably a human) by treating (e.g. alleviating or improving) one or more core features of ASD and by treating (e.g. reducing) agitation and aggression, the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of ASD by treating (e.g. alleviating or improving) one or more core features of ASD and by treating (e.g. reducing) agitation and aggression.

In some embodiments, the present invention provides the use of vafidemstat or a pharmaceutically acceptable salt or solvate thereof for the treatment of ASD by treating (e.g. alleviating or improving) one or more core features of ASD and by treating (e.g. reducing) agitation and aggression.

In some embodiments, the present invention provides vafidemstat or a pharmaceutically acceptable salt or solvate thereof for use in the treatment of ASD by treating (e.g. alleviating or improving) one or more non-aggressive symptoms of ASD and by treating (e.g. reducing) agitation and aggression.

In some embodiments, the present invention provides a method for treating ASD in a patient (preferably a human) by treating (e.g. alleviating or improving) one or more non-aggressive symptoms of ASD and by treating (e.g. reducing) agitation and aggression, the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of ASD by treating (e.g. alleviating or improving) one or more non-aggressive symptoms of ASD and by treating (e.g. reducing) agitation and aggression.

In some embodiments, the present invention provides the use of vafidemstat or a pharmaceutically acceptable salt or solvate thereof for the treatment of ASD by treating (e.g. alleviating or improving) one or more non-aggressive symptoms of ASD and by treating (e.g. reducing) agitation and aggression.

In some embodiments, the present invention provides vafidemstat or a pharmaceutically acceptable salt or solvate thereof for use in the treatment of an ASD patient by treating (e.g. alleviating or improving) one or more core features of ASD.

In some embodiments, the present invention provides a method for treating an ASD patient (preferably a human) by treating (e.g. alleviating or improving) one or more core features of ASD, the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of an ASD patient by treating (e.g. alleviating or improving) one or more core features of ASD.

In some embodiments, the present invention provides the use of vafidemstat or a pharmaceutically acceptable salt or solvate thereof for the treatment of an ASD patient by treating (e.g. alleviating or improving) one or more core features of ASD.

In some embodiments, the present invention provides vafidemstat or a pharmaceutically acceptable salt or solvate thereof for use in the treatment of an ASD patient by treating (e.g. alleviating or improving) one or more non-aggressive symptoms of ASD.

In some embodiments, the present invention provides a method for treating an ASD patient (preferably a human) by treating (e.g. alleviating or improving) one or more non-aggressive symptoms of ASD, the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of an ASD patient by treating (e.g. alleviating or improving) one or more non-aggressive symptoms of ASD.

In some embodiments, the present invention provides the use of vafidemstat or a pharmaceutically acceptable salt or solvate thereof for the treatment of an ASD patient by treating (e.g. alleviating or improving) one or more non-aggressive symptoms of ASD.

In some embodiments, the present invention provides vafidemstat or a pharmaceutically acceptable salt or solvate thereof for use in the treatment of an ASD patient by treating (e.g. alleviating or improving) one or more core features of ASD and by treating (e.g. reducing) aggression.

In some embodiments, the present invention provides a method for treating an ASD patient (preferably a human) by treating (e.g. alleviating or improving) one or more core features of ASD and by treating (e.g. reducing) aggression, the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of an ASD patient by treating (e.g. alleviating or improving) one or more core features of ASD and by treating (e.g. reducing) aggression.

In some embodiments, the present invention provides the use of vafidemstat or a pharmaceutically acceptable salt or solvate thereof for the treatment of an ASD patient by treating (e.g. alleviating or improving) one or more core features of ASD and by treating (e.g. reducing) aggression.

In some embodiments, the present invention provides vafidemstat or a pharmaceutically acceptable salt or solvate thereof for use in the treatment of an ASD patient by treating (e.g. alleviating or improving) one or more non-aggressive symptoms of ASD and by treating (e.g. reducing) aggression.

In some embodiments, the present invention provides a method for treating an ASD patient (preferably a human) by treating (e.g. alleviating or improving) one or more non-aggressive symptoms of ASD and by treating (e.g. reducing) aggression, the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of an ASD patient by treating (e.g. alleviating or improving) one or more non-aggressive symptoms of ASD and by treating (e.g. reducing) aggression.

In some embodiments, the present invention provides the use of vafidemstat or a pharmaceutically acceptable salt or solvate thereof for the treatment of an ASD patient by treating (e.g. alleviating or improving) one or more non-aggressive symptoms of ASD and by treating (e.g. reducing) aggression.

In some embodiments, the present invention provides vafidemstat or a pharmaceutically acceptable salt or solvate thereof for use in the treatment of an ASD patient by treating (e.g. alleviating or improving) one or more core features of ASD and by treating (e.g. reducing) agitation.

In some embodiments, the present invention provides a method for treating an ASD patient (preferably a human) by treating (e.g. alleviating or improving) one or more core features of ASD and by treating (e.g. reducing) agitation, the method comprising administering to the patient a therapeutically effective amount of vafidemstat or a pharmaceutically acceptable salt or solvate thereof

In some embodiments, the present invention provides the use of vafidemstat or a pharmaceutically acceptable salt or solvate thereof for the manufacture of a medicament for the treatment of an ASD patient by treating (e.g. alleviating or improving) one or more core features of ASD and by treating (e.g. reducing) agitation.

In some embodiments, the present invention provides the use of vafidemstat or a pharmaceutically acceptable salt or solvate thereof for the treatment of an ASD patient by treating (e.g. alleviating or improving) one or more core features of ASD and by treating (e.g. reducing) agitation.

In some embodiments, the present invention provides vafidemstat or a pharmaceutically acceptable salt or solvate thereof for use in the treatment of an ASD patient by treating (e.g. alleviating or improving) one or more non-aggressive symptoms of ASD and by treating (e.g. reducing) agitation.

In some embodiments, the present invention provides a method for treating an ASD patient (preferably a human) by treating (e.g. alleviating or improving) one or more non-aggressive symptoms of ASD and by treating (e.g. reducing) agitation, the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of an ASD patient by treating (e.g. alleviating or improving) one or more non-aggressive symptoms of ASD and by treating (e.g. reducing) agitation.

In some embodiments, the present invention provides the use of vafidemstat or a pharmaceutically acceptable salt or solvate thereof for the treatment of an ASD patient by treating (e.g. alleviating or improving) one or more non-aggressive symptoms of ASD and by treating (e.g. reducing) agitation.

In some embodiments, the present invention provides vafidemstat or a pharmaceutically acceptable salt or solvate thereof for use in the treatment of an ASD patient by treating (e.g. alleviating or improving) one or more core features of ASD and by treating (e.g. reducing) agitation and aggression.

In some embodiments, the present invention provides a method for treating an ASD patient (preferably a human) by treating (e.g. alleviating or improving) one or more core features of ASD and by treating (e.g. reducing) agitation and aggression, the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of an ASD patient by treating (e.g. alleviating or improving) one or more core features of ASD and by treating (e.g. reducing) agitation and aggression.

In some embodiments, the present invention provides the use of vafidemstat or a pharmaceutically acceptable salt or solvate thereof for the treatment of an ASD patient by treating (e.g. alleviating or improving) one or more core features of ASD and by treating (e.g. reducing) agitation and aggression.

In some embodiments, the present invention provides vafidemstat or a pharmaceutically acceptable salt or solvate thereof for use in the treatment of an ASD patient by treating (e.g. alleviating or improving) one or more non-aggressive symptoms of ASD and by treating (e.g. reducing) agitation and aggression.

In some embodiments, the present invention provides a method for treating an ASD patient (preferably a human) by treating (e.g. alleviating or improving) one or more non-aggressive symptoms of ASD and by treating (e.g. reducing) agitation and aggression, the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of an ASD patient by treating (e.g. alleviating or improving) one or more non-aggressive symptoms of ASD and by treating (e.g. reducing) agitation and aggression.

In some embodiments, the present invention provides the use of vafidemstat or a pharmaceutically acceptable salt or solvate thereof for the treatment of an ASD patient by treating (e.g. alleviating or improving) one or more non-aggressive symptoms of ASD and by treating (e.g. reducing) agitation and aggression.

In some embodiments, the present invention provides vafidemstat or a pharmaceutically acceptable salt or solvate thereof for use in the treatment of one or more core features of ASD.

In some embodiments, the present invention provides a method for treating one or more core features of ASD in a patient (preferably a human), the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of one or more core features of ASD.

In some embodiments, the present invention provides the use of vafidemstat or a pharmaceutically acceptable salt or solvate thereof for the treatment of one or more core features of ASD.

In some embodiments, the present invention provides vafidemstat or a pharmaceutically acceptable salt or solvate thereof for use in the treatment of one or more non-aggressive symptoms of ASD.

In some embodiments, the present invention provides a method for treating one or more non-aggressive symptoms of ASD in a patient (preferably a human), comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of one or more non-aggressive symptoms of ASD.

In some embodiments, the present invention provides the use of vafidemstat or a pharmaceutically acceptable salt or solvate thereof for the treatment of one or more non-aggressive symptoms of ASD.

In some embodiments, the present invention provides vafidemstat or a pharmaceutically acceptable salt or solvate thereof for use in the treatment of one or more core features of ASD as well as agitation and/or aggression.

In some embodiments, the present invention provides a method for treating one or more core features of ASD as well as agitation and/or aggression in a patient (preferably a human), the method comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of one or more core features of ASD as well as agitation and/or aggression.

In some embodiments, the present invention provides the use of vafidemstat or a pharmaceutically acceptable salt or solvate thereof for the treatment of one or more core features of ASD as well as agitation and/or aggression.

In some embodiments, the present invention provides vafidemstat or a pharmaceutically acceptable salt or solvate thereof for use in the treatment of one or more non-aggressive symptoms of ASD as well as agitation and/or aggression.

In some embodiments, the present invention provides a method for treating one or more non-aggressive symptoms of ASD as well as agitation and/or aggression in a patient (preferably a human), comprising administering to the patient a therapeutically effective amount of vafidemstat or a pharmaceutically acceptable salt or solvate thereof.

In some embodiments, the present invention provides the use of vafidemstat or a pharmaceutically acceptable salt or solvate thereof for the manufacture of a medicament for the treatment of one or more non-aggressive symptoms of ASD as well as agitation and/or aggression.

In some embodiments, the present invention provides the use of vafidemstat or a pharmaceutically acceptable salt or solvate thereof for the treatment of one or more non-aggressive symptoms of ASD as well as agitation and/or aggression.

Also provided herein is vafidemstat or a pharmaceutically acceptable salt or solvate thereof for use in the treatment (e.g. reduction) of agitation in ASD. Likewise provided herein is vafidemstat or a pharmaceutically acceptable salt or solvate thereof for use in the treatment (e.g. reduction) of agitation in an ASD patient. Further provided herein is vafidemstat or a pharmaceutically acceptable salt or solvate thereof for use in the treatment of an ASD patient by treating (e.g. reducing) agitation. Provided herein is furthermore a method for treating (e.g., reducing) agitation in an ASD patient (preferably a human), comprising administering to the patient a therapeutically effective amount of vafidemstat or a pharmaceutically acceptable salt or solvate thereof. Likewise provided herein is the use of vafidemstat or a pharmaceutically acceptable salt or solvate thereof for the manufacture of a medicament for the treatment (e.g. reduction) of agitation in ASD. Further provided herein is the use of vafidemstat or a pharmaceutically acceptable salt or solvate thereof for the treatment (e.g. reduction) of agitation in ASD.

Moreover, provided herein is also vafidemstat or a pharmaceutically acceptable salt or solvate thereof for use in the treatment (e.g. reduction) of aggression in ASD. Likewise provided herein is vafidemstat or a pharmaceutically acceptable salt or solvate thereof for use in the treatment (e.g. reduction) of aggression in an ASD patient. Further provided herein is vafidemstat or a pharmaceutically acceptable salt or solvate thereof for use in the treatment of an ASD patient by treating (e.g. reducing) aggression. Further provided herein is a method for treating (e.g., reducing) aggression in an ASD patient (preferably a human), comprising administering to the patient a therapeutically effective amount of vafidemstat or a pharmaceutically acceptable salt or solvate thereof. Likewise provided herein is the use of vafidemstat or a pharmaceutically acceptable salt or solvate thereof for the manufacture of a medicament for the treatment (e.g. reduction) of aggression in ASD. Provided herein is furthermore the use of vafidemstat or a pharmaceutically acceptable salt or solvate thereof for the treatment (e.g. reduction) of aggression in ASD.

Vafidemstat is the compound 5-((((1R,2S)-2-(4-(benzyloxy)phenyl)cyclopropyl)amino)methyl)-1,3,4-oxadiazol-2-amine, also known as (41R,42S)-6-oxa-3-aza-1(2)-[1,3,4]oxadiazola-5(1,4),8(1)-dibenzena-4(1,2)-cyclopropanaoctaphan-15-amine, or ORY-2001. The names "vafidemstat", "5-((((1R,2S)-2-(4-(benzyloxy)phenyl)cyclopropyl)amino)methyl)-1,3,4-oxadiazol-2-amine", "(41R,42S)-6-oxa-3-aza-1(2)-[1,3,4]oxadiazola-5(1,4),8(1)-dibenzena-4(1,2)-cyclopropanaoctaphan-15-amine" or "ORY-2001" are used herein interchangeably.

In some embodiments, the ASD is adult ASD.

Preferably, vafidemstat (or a pharmaceutically acceptable salt or solvate thereof) is administered orally. Exemplary formulations which can be administered via peroral ingestion are described in more detail further below.

As explained above, the present invention provides the compound vafidemstat, or a pharmaceutically acceptable salt or solvate thereof, for use in the treatment of ASD. Accordingly, the invention relates to the compound vafidemstat as a free base (in non-salt form) for use in the treatment of ASD and, furthermore, the invention also relates to a pharmaceutically acceptable salt or solvate of vafidemstat for use in the treatment of ASD. It is however preferred that the compound is vafidemstat (in non-salt form).

In some embodiments, vafidemstat is administered orally at a dose of 1.2 mg/day in a 5 days on/2 days off schedule.

As illustrated in the Examples, it has been unexpectedly found in the context of the present invention that vafidemstat is useful to treat ASD, such as adult ASD. As part of a Phase Ila clinical trial evaluating vafidemstat as a treatment for aggression in human patients with a range of CNS disorders, it has been shown that vafidemstat produces a significant improvement on aggressive behavior in ASD patients. As illustrated in Example 2 and Figures 1, 2 and 3, treatment with vafidemstat causes an improvement in several commonly used scales to assess aggression, in particular the NPI A/A (Figure 1), the CGI-I (Figure 2) and the CGI-S (Figure 3), in ASD patients after 8 weeks of treatment. The Neuropsychiatric Inventory Agitation/Aggression (NPI A/A) subscale is based on the NPI scale and consists of the 4 items in the NPI related to aggression/agitation, namely: agitation/aggression, disinhibition, irritability and aberrant motor disturbance. The Clinical Global Impression of Improvement (CGI-I) and Clinical Global Impression of Severity (CGI-S) values reflect physician's ratings of patient's improvement in aggression (CGI-I) and patient's aggression severity (CGI-S). As explained in greater detail in Example 2 and illustrated in Figures 4 and 5, it has been surprisingly found that treatment with vafidemstat also produces therapeutic effects on ASD independent to or beyond its effects on aggression. Vafidemstat produced a statistically significant improvement on the Total NPI score comparing the Total NPI score after 8 weeks of treatment with vafidemstat (score at visit 7) with the score at baseline, prior to starting treatment with vafidemstat (score at visit 1), as shown in Figure 4. The total NPI scale consists of 12 items relating to a variety of neuropsychiatric domains and can be used to measure patient's global functioning. In addition to the reduction in the total NPI score, vafidemstat also produced a statistically significant improvement on the NPI non-aggression related combined score comparing the NPI non-aggression related combined score after 8 weeks of treatment with vafidemstat (score at visit 7) with the score at baseline, prior to starting treatment with vafidemstat (score at visit 1), see Figure 5. The NPI non-aggression related combined score is the NPI combined score for all other items in the NPI not related to aggression or agitation (8 items). The results obtained in ASD patients using total NPI scale and non-aggression related NPI subscale show that vafidemstat has a broad therapeutic effect in ASD, with therapeutic effects to treat ASD beyond treating aggresion. Vafidemstat has been found to be particularly well suited to treat ASD, including treating core features of ASD as defined herein.

Importantly, the therapeutic effects of vafidemstat (or a pharmaceutically acceptable salt or solvate thereof) in the treatment of ASD are attainable without producing sedative effects or weight gain.

### Pharmaceutical Formulations

While it is possible that vafidemstat may be administered for use in therapy directly as such, it is typically administered in the form of a pharmaceutical composition, which comprises the compound as active pharmaceutical ingredient together with one or more pharmaceutically acceptable excipients or carriers.

Any reference to vafidemstat throughout this specification includes a reference to the compound as such, i.e. the compound in non-salt form (e.g., as a free base) or in the form of any pharmaceutically acceptable salt or solvate thereof, as well as a reference to a pharmaceutical composition comprising said compound and one or more pharmaceutically acceptable excipients or carriers.

Vafidemstat may be administered by any means that accomplish the intended purpose. Examples include administration by the oral, parenteral (including e.g. intravenous, subcutaneous or intracerebral), or topical routes.

For oral delivery, the compound can be incorporated into a formulation that includes pharmaceutically acceptable carriers such as binders (e.g., gelatin, cellulose, gum tragacanth), excipients (e.g., starch, lactose), lubricants (e.g., magnesium stearate, silicon dioxide), disintegrating agents (e.g., alginate, Primogel, and corn starch), and sweetening or flavoring agents (e.g., glucose, sucrose, saccharin, methyl salicylate, and peppermint). The formulation can be orally delivered, e.g., in the form of enclosed gelatin capsules or compressed tablets. Capsules and tablets can be prepared by any conventional techniques. The capsules and tablets can also be coated with various coatings known in the art to modify the flavors, tastes, colors, and shapes of the capsules and tablets. In addition, liquid carriers such as fatty oil can also be included in capsules.

Suitable oral formulations can also be in the form of suspension, syrup, chewing gum, wafer, elixir, and the like. If desired, conventional agents for modifying flavors, tastes, colors, and shapes of the special forms can also be included. In addition, for convenient administration by enteral feeding tube in patients unable to swallow, the active compounds can be dissolved in an acceptable lipophilic vegetable oil vehicle such as olive oil, corn oil and safflower oil.

The compound can also be administered parenterally in the form of solution or suspension, or in lyophilized form capable of conversion into a solution or suspension form before use. In such formulations, diluents or pharmaceutically acceptable carriers such as sterile water and physiological saline buffer can be used. Other conventional solvents, pH buffers, stabilizers, anti-bacteria agents, surfactants, and antioxidants can all be included. For example, useful components include sodium chloride, acetates, citrates or phosphates buffers, glycerin, dextrose, fixed oils, methyl parabens, polyethylene glycol, propylene glycol, sodium bisulfate, benzyl alcohol, ascorbic acid, and the like. The parenteral formulations can be stored in any conventional containers such as vials and ampoules.

For topical administration, the compound can be formulated into lotions, creams, ointments, gels, powders, pastes, sprays, suspensions, drops and aerosols. Thus, one or more thickening agents, humectants, and stabilizing agents can be included in the formulations. Examples of such agents include, but are not limited to, polyethylene glycol, sorbitol, xanthan gum, petrolatum, beeswax, or mineral oil, lanolin, squalene, and the like. A special form of topical administration is delivery by a transdermal patch. Methods for preparing transdermal patches are disclosed, e.g., in Brown, et al. (1988) Ann. Rev. Med. 39:221-229 which is incorporated herein by reference.

Subcutaneous implantation for sustained release of the compound may also be a suitable route of administration. This entails surgical procedures for implanting an active compound in any suitable formulation into a subcutaneous space, e.g., beneath the anterior abdominal wall. See, e.g., Wilson et al. (1984) J. Clin. Psych. 45:242-247. Hydrogels can be used as a carrier for the sustained release of active compounds. Hydrogels are generally known in the art. They are typically made by crosslinking high molecular weight biocompatible polymers into a network, which swells in water to form a gel like material. Preferably, hydrogels are biodegradable or biosorbable. For purposes of this invention, hydrogels made of polyethylene glycols, collagen, or poly(glycolic-co-L-lactic acid) may be useful. See, e.g., Phillips et al. (1984) J. Pharmaceut. Sci., 73: 1718-1720.

The compound can also be conjugated to a water soluble non-immunogenic non-peptidic high molecular weight polymer to form a polymer conjugate. For example, the compound can be covalently linked to polyethylene glycol to form a conjugate. Typically, such a conjugate exhibits improved solubility, stability, and reduced toxicity and immunogenicity. Thus, when administered to a patient, the compound in the conjugate can have a longer half-life in the body, and exhibit better efficacy. See generally, Burnham (1994) Am. J. Hosp. Pharm. 15:210-218. PEGylated proteins are currently being used in protein replacement therapies and for other therapeutic uses. For example, PEGylated interferon (PEG-INTRON A^{®}) is clinically used for treating Hepatitis B. PEGylated adenosine deaminase (ADAGEN^{®}) is being used to treat severe combined immunodeficiency disease (SCIDS). PEGylated L-asparaginase (ONCAPSPAR^{®}) is being used to treat acute lymphoblastic leukemia (ALL). **It is** preferred that the covalent linkage between the polymer and the active compound and/or the polymer itself is hydrolytically degradable under physiological conditions. Such conjugates known as "prodrugs" can readily release the active compound inside the body. Controlled release of an active compound can also be achieved by incorporating the active ingredient into microcapsules, nanocapsules, or hydrogels generally known in the art. Other pharmaceutically acceptable prodrugs of the compound include, but are not limited to, esters, carbonates, thiocarbonates, N-acyl derivatives, N-acyloxyalkyl derivatives, quaternary derivatives of tertiary amines, N-Mannich bases, Schiff bases, amino acid conjugates, phosphate esters, metal salts and sulfonate esters.

Liposomes can also be used as carriers for the active compound. Liposomes are micelles made of various lipids such as cholesterol, phospholipids, fatty acids, and derivatives thereof. Various modified lipids can also be used. Liposomes can reduce the toxicity of the active compounds, and increase their stability. Methods for preparing liposomal suspensions containing active ingredients therein are generally known in the art. See, e.g., U.S. Patent No. 4,522,811; Prescott, Ed., Methods in Cell Biology, Volume XIV, Academic Press, New York, N. Y. (1976).

The pharmaceutical compositions, like oral and parenteral compositions, can be formulated in unit dosage forms for ease of administration and uniformity of dosage. As used herein, "unit dosage forms" refers to physically discrete units suitable as unitary dosages for administration to subjects, each unit containing a predetermined quantity of active ingredient calculated to produce the desired therapeutic effect, in association with one or more suitable pharmaceutical carriers.

In therapeutic applications, pharmaceutical compositions are to be administered in a manner appropriate to the disease to be treated, as determined by a person skilled in the medical arts. An appropriate dose and suitable duration and frequency of administration will be determined by such factors as the condition of the patient, the type and severity of the disease, the particular form of the active ingredient, the method of administration, among others. In general, an appropriate dose and administration regimen provides the pharmaceutical composition in an amount sufficient to provide therapeutic benefit, for example an improved clinical outcome, such as more frequent complete or partial remissions, or longer disease-free and/or overall survival, or lessening of symptoms severity, or any other objetively identifiable improvement as noted by the clinician. Effective doses may generally be assessed or extrapolated using experimental models like dose-response curves derived from in vitro or animal model test systems, or from clinical trials.

The pharmaceutical compositions of the invention can be included in a container, pack or dispenser together with instructions for administration.

Vafidemstat, have been found to be orally active and to be effective in the treatment of ASD when administered orally, as illustrated in Example 2. Accordingly, it is preferred that vafidemstat is administered by the oral route for the treatment of ASD.

The present invention also embraces the use of vafidemstat, in which one or more atoms are replaced by a specific isotope of the corresponding atom. For example, the invention encompasses the use of vafidemstat, in which one or more hydrogen atoms (or, e.g., all hydrogen atoms) are replaced by deuterium atoms (i.e., ²H; also referred to as "D"). Accordingly, the invention also embraces vafidemstat which is enriched in deuterium. Naturally occurring hydrogen is an isotopic mixture comprising about 99.98 mol-% hydrogen-1 (¹H) and about 0.0156 mol-% deuterium (²H or D). The content of deuterium in one or more hydrogen positions in vafidemstat can be increased using deuteration techniques known in the art. For example, vafidemstat or a reactant or precursor to be used in the synthesis of vafidemstat can be subjected to an H/D exchange reaction using, e.g., heavy water (D₂O). Further suitable deuteration techniques are described in: Atzrodt J et al., Bioorg Med Chem, 20(18), 5658-5667, 2012; William JS et al., Journal of Labelled Compounds and Radiopharmaceuticals, 53(11-12), 635-644, 2010; Modvig A et al., J Org Chem, 79, 5861-5868, 2014. The content of deuterium can be determined, e.g., using mass spectrometry or NMR spectroscopy. Unless specifically indicated otherwise, it is preferred that vafidemstat to be used in accordance with the present invention is not enriched in deuterium. Accordingly, the presence of naturally occurring hydrogen atoms or ¹H hydrogen atoms in vafidemstat is preferred. In general, it is preferred that none of the atoms in vafidemstat to be used in accordance with the invention are replaced by specific isotopes.

### Definitions

Unless defined otherwise, all technical and scientific terms used herein have the same meaning as commonly understood by one of ordinary skill in the art to which this invention pertains.

The following definitions apply throughout the present specification and claims, unless specifically indicated otherwise.

A "patient" or "subject" for the purposes of the present invention includes both humans and other animals, particularly mammals. Thus, the methods and uses of the invention are applicable to both human therapy and veterinary applications. In a preferred aspect the subject or patient is a mammal, and in the most preferred aspect the subject or patient is a human (e.g. a male or female human).

The terms "treatment", "treating" and the like are used herein to generally mean obtaining a desired pharmacological and/or physiological effect. The effect may be prophylactic in terms of completely or partially preventing a disease (herein, ASD) or symptom thereof and/or may be therapeutic in terms of partially or completely curing or ameliorating a disease (i.e. ASD) and/or a symptom or adverse effect attributed to the disease or partially or completely halting the progression of a disease and/or a symptom or adverse effect attributed to the disease. The term "treatment" as used herein covers any treatment of a disease (i.e. ASD) in a patient and includes, without limitation, any one or more of the following: (a) preventing ASD in a patient which may be predisposed/at risk of developing ASD; (b) delaying the onset of ASD; (c) inhibiting ASD, i.e. arresting, delaying or slowing down its development/progression; or (d) relieving the ASD, i.e. causing (complete or partial) regression, correction or alleviation of ASD. The present invention specifically and distinctly relates to each one of these forms of treatment.

As used herein, the term "therapeutically effective amount" refers to the amount sufficient to produce a desired biological effect (e.g., a therapeutic effect) in a subject. Accordingly, a therapeutically effective amount of a compound may be an amount which is sufficient to treat a disease (i.e. ASD), and/or delay the onset or progression of the disease, and/or alleviate one or more symptoms of the disease, when administered to a subject suffering from or susceptible to that disease.

As used herein, the abbreviation "ASD" refers to autism spectrum disorder.

As used herein, a "pharmaceutically acceptable salt" is intended to mean a salt that retains the biological effectiveness of the free acids and/or bases of the specified compound and that is not biologically or otherwise undesirable. A compound may possess a sufficiently acidic, a sufficiently basic, or both functional groups, and accordingly react with any of a number of inorganic or organic bases, and inorganic and organic acids, to form a pharmaceutically acceptable salt. Exemplary pharmaceutically acceptable salts include those salts prepared by reaction of a compound according to the invention, e.g. vafidemstat with a mineral or organic acid, such as hydrochlorides, hydrobromides, sulfates, pyrosulfates, bisulfates, sulfites, bisulfites, phosphates, monohydrophosphates, dihydrophosphates, metaphosphates, pyrophosphates, chlorides, bromides, iodides, nitrates, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, gamma-hydroxybutyrates, glycollates, tartrates, methanesulfonates, ethane-sulfonates, propanesulfonates, benzenesulfonates, toluenesulfonates, trifluoromethansulfonates, naphthalene-1-sulfonates, naphthalene-2-sulfonates, mandelates, pyruvates, stearates, ascorbates, or salicylates. When a compound carries an acidic moiety, suitable pharmaceutically acceptable salts thereof may include alkali metal salts, e.g. sodium or potassium salts; alkaline earth metal salts, e.g. calcium or magnesium salts; and salts formed with suitable organic ligands such as ammonia, alkylamines, hydroxyalkylamines, lysine, arginine, N-methylglucamine, procaine and the like. Pharmaceutically acceptable salts are well known in the art.

As used herein, a "pharmaceutically acceptable solvate" refers to a complex of variable stoichiometry formed by a solute and a pharmaceutically acceptable solvent such as water, ethanol and the like. A complex with water is known as a hydrate. It is to be understood that the invention encompasses pharmaceutically acceptable solvates of vafidemstat in non-salt form and also in the form of a pharmaceutically acceptable salt thereof.

As used herein, a "pharmaceutically acceptable carrier" or "pharmaceutically acceptable excipient" refers to non-API (API refers to Active Pharmaceutical Ingredient) substances such as disintegrators, binders, fillers, and lubricants used in formulating pharmaceutical products. They are generally safe for administering to humans according to established governmental standards, including those promulgated by the United States Food and Drug Administration and/or the European Medicines Agency. Pharmaceutically acceptable carriers or excipients are well known to those skilled in the art.

As used herein, the term "comprising" (or "comprise", "comprises", "contain", "contains", or "containing"), unless explicitly indicated otherwise or contradicted by context, has the meaning of "containing, inter alia", i.e., "containing, among further optional elements, ...". In addition thereto, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes the meaning of "A consisting essentially of B and C" and the meaning of "A consisting of B and C" (i.e., no other components than B and C are comprised in A).

As used herein, unless explicitly indicated otherwise or contradicted by context, the terms "a", "an" and "the" are used interchangeably with "one or more" and "at least one".

### EXAMPLES

The following examples illustrate various aspects of the invention. The examples should, of course, be understood to be merely illustrative of only certain embodiments of the invention and not to constitute limitations upon the scope of the invention. Results are also presented and described in the Figures and Figure legends.

### Example 1: Vafidemstat

Vafidemstat (recommended International Nonproprietary Name) is the compound 5-((((1R,2S)-2-(4-(benzyloxy)phenyl)cyclopropyl)amino)methyl)-1,3,4-oxadiazol-2-amine, also known as (-) 5-((((trans)-2-(4-(benzyloxy)phenyl)cyclopropyl)amino)methyl)-1,3,4-oxadiazol-2-amine, (41R,42S)-6-oxa-3-aza-1(2)-[1,3,4]oxadiazola-5(1,4),8(1)-dibenzena-4(1,2)-cyclopropanaoctaphan-15-amine or ORY-2001, and whose chemical structure is shown below.

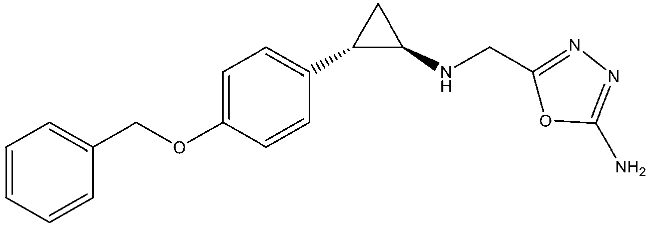

This compound can be obtained as disclosed in WO2012/013728.

Vafidemstat is a KDM1A inhibitor, with a mean IC₅₀ value of 101 ± 40 nM obtained in the KDM1A assay described below.

*In vitro KDM1A inhibition assay:* Human recombinant KDM1A protein (GenBank accession no. NM_015013, amino acids 158-end with N-terminal GST tag, MW: 103 kDa) was used. Serial 3-fold dilutions of test compound ranged between 30 µM and 1 nM were pre-incubated for 15 min with human recombinant KDM1A enzyme (BPS Bioscience, Ref. 50100) on ice in the assay buffer (50 mM sodium phosphate pH 7.4). Each concentration of inhibitor was tested in duplicate. The enzymatic reaction was initiated by the addition of dimethyl H3K4 peptide substrate (Anaspec, Ref. 63677), at the appK_{M} of KDM1A. After 30 min of incubation at 37°C Amplex Red reagent and the horseradish peroxidase (HRP) solution were added to detect H₂O₂ formed in the enzymatic reaction, following the recommendations provided by the supplier (Invitrogen). The mix was incubated for 5 min at room temperature in the dark and the conversion of the Amplex Red reagent to the highly fluorescent resorufin was analyzed using an Infinite F200 Tecan fluorescence microplate reader (λexcitation=540 nm, λemission=590 nm). The maximum demethylase activity of KDM1A was obtained in the absence of inhibitor and corrected for background fluorescence in the absence of KDM1A. The IC₅₀ value for test compound was calculated with GraphPad Prism5 Software from a minimum of two independent experiments.

### Example 2: Evaluation of the effect of vafidemstat to treat ASD in humans

As part of a Phase IIa clinical trial (REIMAGINE trial, EudraCT number 2018-002140-88) to evaluate the safety, tolerability and efficacy of vafidemstat to treat aggression in adult population in patients with different CNS disorders, a cohort of ASD patients was recruited and treated with vafidemstat for 8 weeks. A summary of the protocol of this clinical trial and results obtained in the ASD cohort are provided below.

### 2.1 Clinical trial design

Reimagine is a unicenter, open-label, 1-arm, 8-week clinical study to evaluate the efficacy, safety and tolerability of vafidemstat in aggression in adult population with ASD, ADHD and BPD.

Main objective of the trial: To evaluate the safety and tolerability of vafidemstat in adult population with ASD ADHD and BPD.

Secondary objectives of the trial: To investigate the efficacy of vafidemstat in aggression in adult population with ASD ADHD and BPD.

Main inclusion criteria:
- age 18-85
- current diagnosis for ASD, ADHD or BPD according to DSM-5 criteria
- significant or persistent agitation or aggression that was disruptive to patient's daily living or put the patient in harm's way for at least 3 days per week for at least 4 weeks prior to screening visit

Treatment: All patients received vafidemstat (as free base) at a dose of 1.2 mg/day, administered orally as a single capsule, in a 5 days on/2 days off schedule, during 8 weeks.

### 2.2 ASD cohort

Six ASD patients were recruited, but there was one drop-out, and therefore the results as described herein correspond to the 5 ASD subjects eligible for analysis. A summary of the patients recruited in this ASD cohort (demographic data at baseline) can be found in Table 1.

**Table 1:**

| **Demographic data ASD patients** | | |
|---|---|---|
| nº of patients | | 6 |
| Sex | Male | 5 (83.3 %) |
| | Female | 1(16.6 %) |
| Age | Median (years) | 35.33 |
| | (Min , Max ) | (24/44) |
| Race | Caucasian | 6(100%) |
| Weight | Median (Kg) | 95.70 |
| | (Min , Max ) | 77.5/116.5 |
| Height | Median (cm) | 181.08 |
| | (Min , Max ) | (174/190) |
| BMI | Median | 29.06 |
| | (Min , Max ) | (22.56/37.46) |

### 2.3 Efficacy assessments in the ASD cohort

Evaluation of the effect of treatment with vafidemstat on aggression was performed using the NPI A/A as well as the CGI-I and CGI-S scales. The NPI A/A subscale is based on the NPI and consists of the 4 items in the NPI related to aggression/agitation, namely: agitation/aggression, disinhibition, irritability and aberrant motor disturbance. The severity of each of the 4 items in the NPI A/A scale is rated from 1 to 3 (1= mild, 2= moderate, 3 = severe) and the NPI A/A score is calculated as the sum of the scores for all 4 items. CGI values reflect clinician's ratings of patient's aggression severity (CGI-S scale) and improvement in aggression from the initiation (baseline) of the treatment (CGI-I scale). In the CGI-I scale, change from initiation of treatment is rated using a seven-point scale, where 1 = very much improved since initiation of treatment, 7= very much worse since initiation of treatment, and 4 = no change from baseline. In the CGI-S scale, illness/condition severity is rated using a seven-point scale, the higher the score, the more severe the illness/condition, with 1 = normal, not at all ill and 7 = among the most extremely ill patients.

In addition to assessing its effect on aggression, the effect of treatment with vafidemstat on ASD patients was also evaluated using the total NPI scale and the NPI non-aggression related combined score. The total NPI scale consists of 12 items relating to a variety of neuropsychiatric domains (namely: delusions, hallucinations, agitation/aggression, depression, anxiety, euphoria/elation, apathy/indifference, disinhibition, irritability, aberrant motor disturbance, nightime behavior, and appetite/eating disorders) and was used to measure global functioning in ASD patients. The severity of each item in the NPI is rated from 1 to 3 (1= mild, 2= moderate, 3 = severe) and the total NPI score is calculated as the sum of the scores for all 12 items. The NPI non-aggression related combined score is the NPI combined score for all other items in the NPI not related to aggression or agitation (8 items; namely: delusions, hallucinations, depression, anxiety, euphoria/elation, apathy/indifference, nightime behavior, and appetite/eating disorders). The severity of each item in the NPI non-aggression related scale is rated from 1 to 3 (1= mild, 2= moderate, 3 = severe) and the NPI non-aggression related combined score is calculated as the sum of the scores for all 8 items.

All efficacy evaluations were performed by assessing the change from baseline (visit 1) to week 8 (visit 7) of the respective scale scores. In the graphs, data is represented as mean ± standard error of the mean (SEM). Statistical analysis was performed using paired one-tail t-test analysis to compare Visit 1 with Visit 7 values. Corresponding p values are indicated in the graphs and in the Results section below. In the case of CGI-S, no paired test could be calculated as all data pairs had the same difference.

### 2.4 Results

Treatment with vafidemstat in ASD patients was safe and well tolerated, without significant adverse events. One patient experienced a transitory event related to hematological alteration, although no clinically relevant modification of the hematological and biochemical parameters tested were observed. No sedation was reported. Treatment of ASD patients with vafidemstat for 8 weeks produced a significant improvement in aggression, as shown by statistically significant reductions in the NPI A/A and CGI-I values from visit 1 to visit 7, as depicted in Figures 1 (NPI A/A, p= 0.0098) and 2 (CGI-I, p= 0.0019). A reduction in the CGI-S value from visit 1 to visit 7 was also observed (see Figure 3). Additional data were obtained within the REIMAGINE trial for one additional ASD patient enrolled in the study, and overall CGI-S data (i.e. n= 6) is presented in Figure 6, showing a statistically significant reduction in the CGI-S score from visit 1 to visit 7 (p= 0.0006).

Unexpectedly, in addition to producing an improvement in aggression, the total NPI score and the NPI non-aggression related combined score also showed a statistically significant reduction after 2 months of treatment with vafidemstat, as shown in Figure 4 (total NPI, p=0.0019) and Figure 5 (non-aggression related NPI, p= 0.0142). These results indicate that vafidemstat exerts therapeutic effects in ASD patients beyond a therapeutic effect on aggression.

In summary, the data and results provided in Example 2 support the use of vafidemstat for the treatment of ASD, including the treatment of ASD core features or ASD symptoms unrelated to aggression.

All publications, patents and patent applications cited herein are hereby incorporated herein by reference in their entireties.

The publications, patents and patent applications mentioned in the specification are provided solely for their disclosure prior to the filing date of the present application. Nothing herein is to be construed as an admission that they are prior art to the instant application.

While the invention has been described in connection with specific embodiments thereof, it will be understood that it is capable of further modifications and this application is intended to cover any variations, uses or adaptations of the invention following, in general, the principles of the invention and including such departures from the present disclosure as come within known or customary practice within the art to which the invention pertains and as may be applied to the essential features hereinbefore set forth and as follows in the appended claims.

The present invention particularly relates to the following items:
1. Vafidemstat or a pharmaceutically acceptable salt or solvate thereof for use in the treatment of autism spectrum disorder.
2. A pharmaceutical composition for use in the treatment of autism spectrum disorder, wherein the pharmaceutical composition comprises vafidemstat or a pharmaceutically acceptable salt or solvate thereof and one or more pharmaceutically acceptable excipients or carriers.
3. The compound for use according to item 1 or the pharmaceutical composition for use according to item 2, wherein the patient to be treated is a human.
4. The compound for use according to any one of items 1 or 3 or the pharmaceutical composition for use according to any one of items 2 or 3, wherein said compound or said pharmaceutical composition is administered orally.
5. The compound for use according to any one of items 1, 3 or 4 or the pharmaceutical composition for use according to any one of items 2 to 4, wherein the autism spectrum disorder is adult autism spectrum disorder.
6. The compound for use according to any one of items 1 or 3 to 5 or the pharmaceutical composition for use according to any one of items 2 to 5, wherein said compound or the compound in said pharmaceutical composition is vafidemstat.
7. A method for treating autism spectrum disorder in a patient, comprising administering to the patient a therapeutically effective amount of vafidemstat or a pharmaceutically acceptable salt or solvate thereof.
8. The method according to item 7, wherein the patient to be treated is a human.
9. The method according to item 7 or 8, wherein the method comprises orally administering vafidemstat or a pharmaceutically acceptable salt or solvate thereof.
10. The method according to any one of items 7 to 9, wherein the autism spectrum disorder is adult autism spectrum disorder.
11. The method according to any one of items 7 to 10, wherein the method comprises administering vafidemstat.
12. Use of vafidemstat or a pharmaceutically acceptable salt or solvate thereof for the manufacture of a medicament for the treatment of autism spectrum disorder.
13. The use according to item 12, wherein the medicament is for the treatment of a human patient.
14. The use according to item 12 or 13, wherein the medicament is for oral administration.
15. The use according to any one of items 12 to 14, wherein the autism spectrum disorder is adult autism spectrum disorder.
16. The use according to any one of items 12 to 15, wherein vafidemstat is used for the manufacture of the medicament.
17. Use of vafidemstat or a pharmaceutically acceptable salt or solvate thereof for the treatment of autism spectrum disorder.
18. The use according to item 17, wherein the patient to be treated is a human.
19. The use according to item 17 or 18, wherein vafidemstat or a pharmaceutically acceptable salt or solvate thereof is administered orally.
20. The use according to any one of items 17 to 19, wherein the autism spectrum disorder is adult autism spectrum disorder.
21. The use according to any one of items 17 to 20, wherein vafidemstat is administered.

## Claims

1. A compound, which is vafidemstat or a pharmaceutically acceptable salt or solvate thereof, for use in the treatment of autism spectrum disorder.

2. A pharmaceutical composition for use in the treatment of autism spectrum disorder, wherein the pharmaceutical composition comprises a compound, which is vafidemstat or a pharmaceutically acceptable salt or solvate thereof, and one or more pharmaceutically acceptable excipients or carriers.

3. The compound for use according to claim 1 or the pharmaceutical composition for use according to claim 2, wherein the patient to be treated is a human.

4. The compound for use according to any one of claims 1 or 3 or the pharmaceutical composition for use according to any one of claims 2 or 3, wherein said compound or said pharmaceutical composition is administered orally.

5. The compound for use according to any one of claims 1, 3 or 4 or the pharmaceutical composition for use according to any one of claims 2 to 4, wherein said compound or the compound in said pharmaceutical composition is vafidemstat.
